# FASCICULE DE BREVET EUROPEEN

(11) **EP 3 873 204 B1**
(45) Date de publication et mention de la délivrance du brevet: **07.06.2023**
(21) Numéro de dépôt: 19816377.6
(22) Date de dépôt: 29.10.2019
(51) Int. Cl.: A01K 67/027, C12N 5/095, C12N 5/09, G01N 33/50

(54) **MODELE ANIMAL POUR AMPLIFIER DES CELLULES TUMORALES CIRCULANTES HUMAINES OU ANIMALES**
TIERMODELL ZUR VERSTÄRKUNG VON MENSCHLICHEN ODER TIERISCHEN ZIRKULIERENDEN TUMORZELLEN
ANIMAL MODEL FOR AMPLIFYING HUMAN OR ANIMAL CIRCULATING TUMOR CELLS

(30) Priorité: 29.10.2018 FR 1859992
(43) Date de publication de la demande: 08.09.2021
(73) Titulaire: INOVOTION, 38700 La Tronche (FR); Hospices Civils de Lyon, 69002 Lyon (FR); Université Claude Bernard Lyon 1, 69100 Villeurbanne (FR)
(72) Inventeur: ROUSSET, Xavier, 38400 Saint Martin D'heres (FR); DOSDA, Emilien, 38850 Chirens (FR); VIALLET, Jean, 38610 Gières (FR); PAYEN-GAY, Léa, 69300 Caluire Et Cuire (FR); MAILLET, Denis, 69001 Lyon (FR)
(74) Mandataire: Regimbeau
(86) Numéro de dépôt international: PCT/FR2019/052571
(87) Numéro de publication internationale: WO 2020/089560

(56) Documents cités:
- WO-A2-2006/001021
- US-B1- 6 228 345
- ELENA I DERYUGINA ET AL: "Chick embryo chorioallantoic membrane model systems to study and visualize human tumor cell metastasis", HISTOCHEMISTRY AND CELL BIOLOGY, SPRINGER, BERLIN, DE, vol. 130, no. 6, 13 novembre 2008 (2008-11-13), pages 1119-1130, XP019657790, ISSN: 1432-119X, DOI: 10.1007/S00418-008-0536-2
- APARNA JAYACHANDRAN ET AL: "Embryonic Chicken Transplantation is a Promising Model for Studying the Invasive Behavior of Melanoma Cells.Neural crest specification: tissues, signals, and transcription factors", FRONTIERS IN ONCOLOGY, vol. 5, 16 février 2015 (2015-02-16), pages 1-7, XP055262052, DOI: 10.3389/fonc.2015.00036
- MANGIR: "An Improved In Vivo Methodology to Visualise Tumour Induced Changes in Vasculature Using the Chick Chorionic Allantoic Membrane Assay", IN VIVO, vol. 32, no. 3, 2 mai 2018 (2018-05-02), XP055584235, DOI: 10.21873/invivo.11262
- DOMENICO RIBATTI: "The chick embryo chorioallantoic membrane as a model for tumor biology", EXPERIMENTAL CELL RESEARCH, vol. 328, no. 2, 1 novembre 2014 (2014-11-01), pages 314-324, XP055584385, AMSTERDAM, NL ISSN: 0014-4827, DOI: 10.1016/j.yexcr.2014.06.010
- SHARMA SANDHYA ET AL: "Circulating tumor cell isolation, culture, and downstream molecular analysis", BIOTECHNOLOGY ADVANCES, ELSEVIER PUBLISHING, BARKING, GB, vol. 36, no. 4, 17 mars 2018 (2018-03-17), pages 1063-1078, XP085400115, ISSN: 0734-9750, DOI: 10.1016/J.BIOTECHADV.2018.03.007
- KHOO BEE LUAN ET AL: "Expansion of patient-derived circulating tumor cells from liquid biopsies using a CTC microfluidic culture device.", NATURE PROTOCOLS JAN 2018, vol. 13, no. 1, janvier 2018 (2018-01), pages 34-58, XP002790927, ISSN: 1750-2799

## Description

### DOMAINE DE L'INVENTION

La présente invention concerne un modèle aviaire permettant l'amplification de cellules tumorales circulantes (CTC) humaines ou animales.

### ART ANTERIEUR

Les CTC sont des cellules qui se détachent d'une tumeur et qui sont capables de se déplacer pour passer dans le sang, après avoir rejoint le système vasculaire. Ces cellules peuvent donc migrer vers d'autres organes et participer au développement de métastases. Ces cellules représentent donc une cible très attractive dans le suivi de l'évolution et du traitement des cancers, voire dans la découverte de nouvelles thérapeutiques.

L'isolement des CTC n'est cependant pas chose aisée car ces cellules sont très peu nombreuses. Il est donc nécessaire de les expandre avant de pouvoir les étudier. Actuellement, les CTC peuvent être amplifiées en utilisant des cultures *in vitro* ou *in vivo* dans des modèles de souris (Kowalik A et al., 2017; Drapkin B. J. et al., 2018). Des cultures *in vitro* de CTC issues de nombreux cancers (cancer du poumon, de la prostate, du sein, des voies urinaires, de la tête et du cou...) ont déjà été réalisées permettent d'effectuer des tests précliniques à haut débit sur des schémas thérapeutiques, mais avec un succès limité car ces cultures sont trop longues pour être compatibles avec la pratique clinique et exposent les CTC à des modifications phénotypiques (Pantel K, Alix-Panabières C., 2016).

Des cultures *in vivo* à court terme ont par ailleurs été établies dans un modèle murin, à partir des CTC, et après une première étape d'expansion *in vitro* lorsque la quantité de CTC était trop faible (Giuliano M et al., 2015 ; Williams ES et al., 2015 ; Torphy RJ et al., 2014 ; Rossi E et al., 2013). L'inoculation directe de CTC à des souris immunodéficientes a également été envisagée comme méthode pour amplifier les CTC isolées. Dans les cancers bronchiques à petites cellules hautement métastatiques (CPPC), des CTC isolées chez des patients présentant un taux élevé de CTC (> 400 CTC par 7,5 mL de sang) ont formé des tumeurs chez les souris dont la réponse aux chimiothérapies au platine et à l'étoposide correspondait aux observations cliniques (Hodgkinson, C. L. et al., 2014). Ces études ouvrent la voie vers l'utilisation des souris immunodéficientes comme "incubateurs" de CTC, susceptibles de générer des modèles précliniques adaptés aux tumeurs individuelles des patients.

Cependant, ces modèles sont trop coûteux pour envisager une utilisation systématique. Ils nécessitent fréquemment de devoir procéder à une étape préalable d'expansion *in vitro* avec les désavantages mentionnés ci-dessus. Enfin, l'établissement de ces modèles nécessite du temps, ce qui n'est pas toujours compatibles avec la constatation des effets souhaités.

Il existe donc un besoin de trouver un modèle *in vivo* moins coûteux, mais également qui donne des résultats plus rapidement, et en particulier qui ne nécessite pas de phase d'expansion *in vitro* préalable afin de travailler sur ces CTC qui sont au plus proche de celles présentes *in vivo* chez les patients.

### RESUME DE L'INVENTION

La présente invention concerne un modèle animal d'étude des cellules tumorales circulantes constitué d'un oeuf embryonné d'oiseau, préférentiellement de poulet, comprenant des cellules tumorales circulantes (CTC) humaines ou animales isolées à partir d'un échantillon d'un patient ou d'un animal atteint de cancer, lesdites CTC étant greffées au niveau de la membrane chorioallantoïque (CAM) d'un oeuf embryonné étant à un stade de développement qui correspond à la formation de la CAM au moment de la greffe et équivalent à au moins 8 jours de développement chez le poulet.

Cet oeuf embryonné est un modèle animal, et plus particulièrement aviaire, d'amplification des CTC. Il permet également d'étudier l'effet de différents agents sur la tumorigenèse pour sélectionner ceux qui possèdent une activité anti-cancéreuse et qui peuvent par conséquent représenter des thérapies efficaces, notamment pour traiter le patient ou l'animal dont sont issues les CTC qui ont été greffées dans l'œuf embryonné. Ceci permet alors d'administrer au patient un agent thérapeutique testé au préalable sur des cellules de sa propre tumeur ; conduisant ainsi à un traitement personnalisé.

La présente invention est également relative à un procédé de préparation de ce modèle d'oeuf embryonné d'oiseau, permettant l'amplification de cellules tumorales circulantes (CTC) humaines ou animales issues d'un échantillon d'un patient ou d'un animal atteint de cancer, ledit procédé comprenant la greffe des CTC isolées à partir dudit échantillon au niveau de la membrane chorioallantoïque (CAM) d'un oeuf embryonné d'oiseau qui est à un stade de développement correspondant à la formation de la CAM et équivalent à au moins 8 jours de développement chez le poulet.

La présente invention a également pour objet un procédé d'amplification de cellules tumorales circulantes (CTC) humaines ou animales isolées à partir d'un échantillon d'un patient ou d'un animal atteint de cancer, comprenant les étapes suivantes :
a) la préparation d'un modèle d'oeuf embryonné conformément à l'invention, par la greffe de CTC isolées à partir d'un échantillon d'un patient ou d'un animal atteint de cancer au niveau de la membrane chorioallantoïque (CAM) d'un oeuf embryonné d'oiseau qui est à un stade de développement correspondant à la formation de la CAM et équivalent à au moins 8 jours de développement chez le poulet,
b) le prélèvement des tumeurs qui se développent à partir des CTC qui se sont amplifiées, et éventuellement
c) la récupération des CTC à partir des tumeurs prélevées à l'étape b). De préférence, plusieurs tours de greffe peuvent être réalisés dans le cadre de ce procédé, et avantageusement deux tours de greffe, afin d'optimiser encore l'amplification des CTC. Il s'agit donc de procéder à une greffe secondaire, sur la CAM d'un deuxième oeuf embryonné, des tumeurs dérivées d'une première greffe de CTC sur la CAM d'un premier oeuf embryonné.

La présente invention est également relative à un procédé de détermination de la sensibilité d'un patient ou d'un animal atteint de cancer à un ou plusieurs agent(s) thérapeutique(s), caractérisé en ce qu'il comprend :
- l'amplification de cellules tumorales circulantes (CTC) issues d'un échantillon dudit patient ou animal atteint de cancer conformément à la présente invention, par la greffe de CTC isolées à partir d'un échantillon d'un patient ou d'un animal atteint de cancer au niveau de la membrane chorioallantoïque (CAM) d'un oeuf embryonné d'oiseau qui est à un stade de développement correspondant à la formation de la CAM et équivalent à au moins 8 jours de développement chez le poulet,
- la greffe des tumeurs qui se sont développées dans l'embryon, au niveau de la membrane chorioallantoïque (CAM) d'un nouvel oeuf embryonné d'oiseau préalablement incubé jusqu'à un stade de développement correspondant à la formation de la CAM et équivalent à au moins 8 jours de développement chez le poulet,
- l'administration du ou des agents thérapeutiques dans l'œuf embryonné au moins 12 heures après la greffe,
- l'étude de l'effet du ou des agents thérapeutiques ainsi administrés sur la tumorigenèse des tumeurs qui se sont développées dans ce nouvel oeuf embryonné greffé.

De préférence, ce procédé de détermination de la sensibilité d'un patient ou d'un animal atteint de cancer à un ou plusieurs agent(s) thérapeutique(s) comprend en outre, après l'administration du ou desdits agents, l'incubation de l'œuf embryonné greffé pendant au moins 1 heure, et éventuellement une étape de prélèvement des tumeurs qui se développent à partir des CTC greffées au terme de l'incubation dudit nouvel oeuf embryonné greffé.

La présente invention a par ailleurs pour objet un procédé de suivi d'un patient ou d'un animal atteint de cancer, comprenant :
- la préparation d'un premier oeuf embryonné d'oiseau comme décrit ci-dessus, avec des CTC isolées à partir d'un échantillon dudit patient ou animal à un instant T1, et l'étude de la tumorigenèse des tumeurs qui se développent dans ce premier oeuf embryonné,
- la préparation d'un second oeuf embryonné d'oiseaux comme décrit ci-dessus, avec des CTC isolées à partir d'un échantillon du même patient ou animal à un instant T2, et l'étude de la tumorigenèse des tumeurs qui se développent dans ce second oeuf embryonné,
- la comparaison de la tumorigenèse des tumeurs qui se sont développées dans le premier et dans le second oeuf embryonné.

Enfin, la présente invention a pour objet un procédé de criblage d'agents thérapeutiques destinés au traitement du cancer *in vivo,* comprenant :
- la préparation d'un modèle d'oeuf embryonné selon le procédé décrit ci-dessus, par la greffe de CTC isolées à partir d'un échantillon d'un patient ou d'un animal atteint de cancer au niveau de la membrane chorioallantoïque (CAM) d'un oeuf embryonné d'oiseau qui est à un stade de développement correspondant à la formation de la CAM et équivalent à au moins 8 jours de développement chez le poulet,
- l'administration d'un ou de plusieurs agents candidats dans l'œuf embryonné au moins 12 heures après la greffe,
- l'étude de l'effet du ou des agents thérapeutiques ainsi administrés sur la tumorigenèse des tumeurs qui se sont développées dans l'œuf embryonné greffé.

### LEGENDES DE FIGURES

La figure 1 représente un schéma du modèle d'oeuf embryonné avec le site de greffe des CTC sur la CAM supérieure.
La figure 2 représente un schéma expérimental possible de réalisation de l'invention :
   Premier tour de greffe (premier groupe d'oeufs) : les CTC isolées à partir d'un échantillon collecté chez le patient sont greffées dans un oeuf par patient. Deuxième tour de greffe (deuxième groupe d'oeufs) : la greffe secondaire permet une nouvelle amplification des tumeurs collectées dans les oeufs post-greffe.

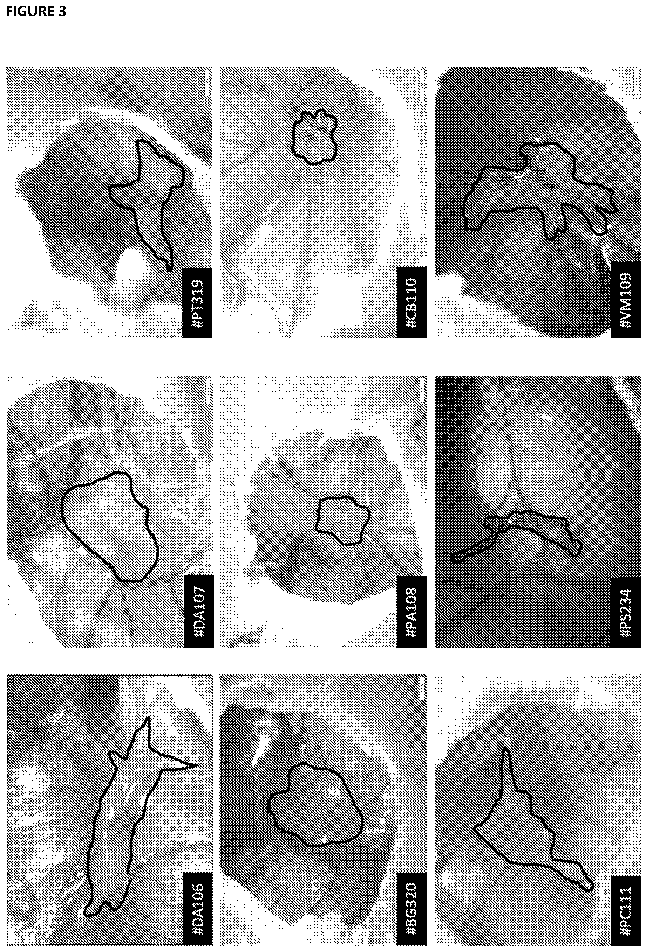

La figure 3 représente des images *in ovo* de croissance de tumeurs obtenues par xénogreffes sur CAM au premier tour d'amplification de CTC isolées à partir de sang de patients atteints de cancer du poumon, de cancer du sein, ou de cancer de la prostate.

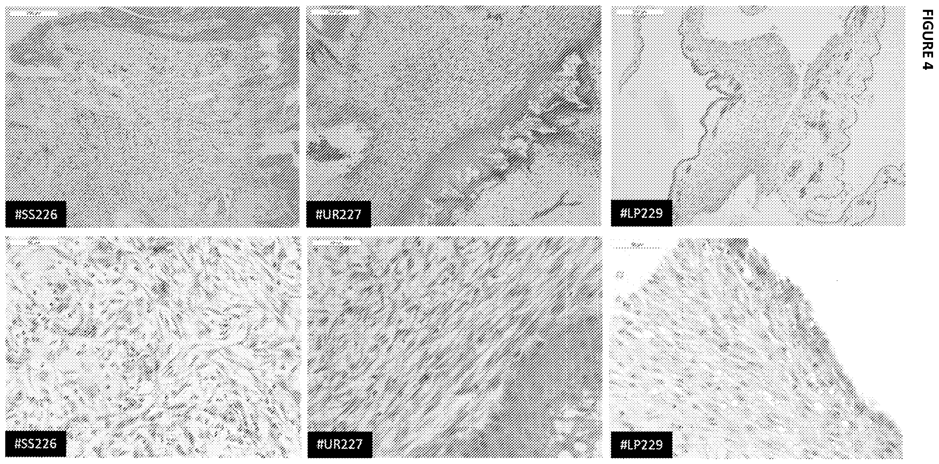

La figure 4 représente des coupes histologiques de tumeurs dérivées de CTC isolées à partir de sang de patientes atteintes du cancer du sein, greffées sur CAM au premier tour d'amplification.

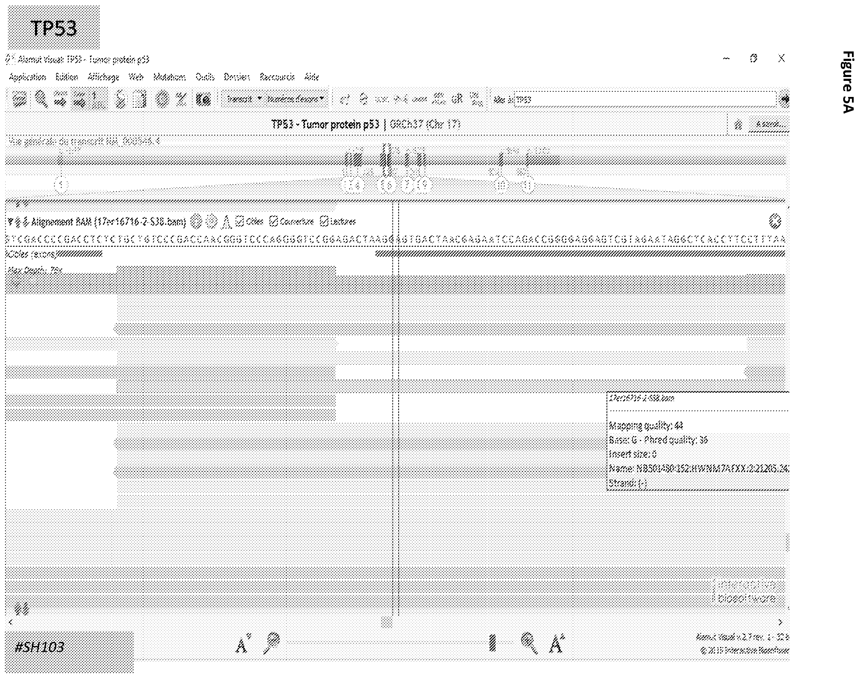

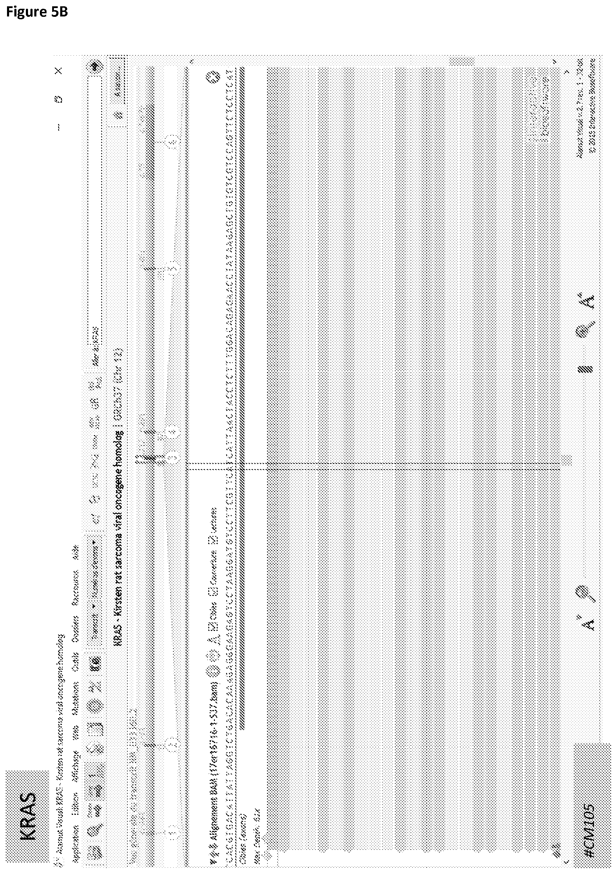

La figure 5 représente les résultats d'analyses génétiques des marqueurs tumoraux humains TP53 (A) et KRAS (B) sur des tumeurs dérivées de CTC de patients atteints de cancer du poumon (#SH103 et #CM105), greffées sur CAM au premier tour d'amplification.

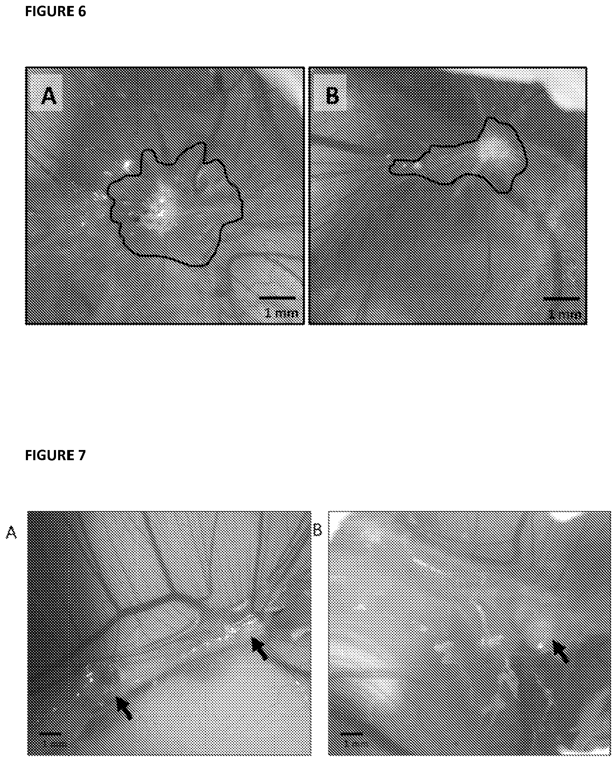

La figure 6 représente des images *in ovo* de croissance de tumeurs dérivées de CTC isolées à partir du sang d'un patient atteint d'un cancer du poumon, greffées sur CAM au second tour d'amplification (greffe secondaire).

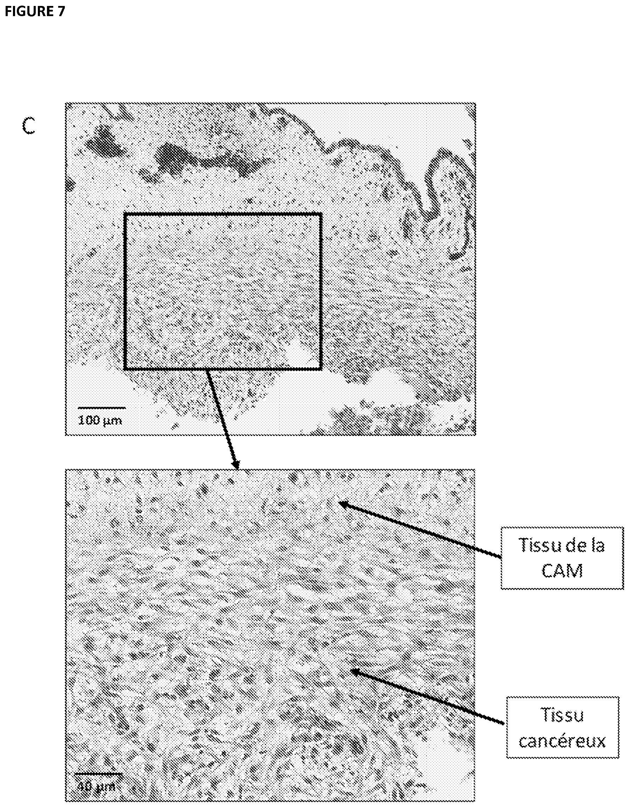

La figure 7 représente les images de tumeurs obtenues *in ovo* à partir de CTC isolées du sang d'un patient atteint d'un cancer de la prostate suite à la greffe primaire (A) et secondaire (B), ainsi que l'analyse histologique d'une coupe de la tumeur (C) démontrant l'amplification des CTC greffées *in ovo* après une greffe secondaire.

### DESCRIPTION DETAILLEE DE L'INVENTION

La présente invention concerne un modèle animal, en particulier aviaire, d'étude des cellules tumorales circulantes constitué d'un d'oeuf embryonné d'oiseau comprenant des cellules tumorales circulantes (CTC) humaines ou animales isolées à partir d'un échantillon d'un patient ou d'un animal atteint de cancer, lesdites CTC étant greffées au niveau de la membrane chorioallantoïque (CAM) d'un oeuf embryonné étant à un stade de développement qui correspond à la formation de la CAM et équivalent à au moins 8 jours de développement chez le poulet au moment de la greffe

De préférence, l'œuf embryonné selon l'invention est un oeuf d'oiseau de l'ordre des Galliformes ou des Struthioniformes. En particulier, il est particulièrement préféré que l'œuf soit un oeuf de gallinacés, et notamment de poulet, caille, dinde, faisan, paon, pintade ou d'autres oiseaux de basse-cour. Il peut également être un oeuf d'autruche. Avantageusement, l'œuf embryonné selon la présente invention est un oeuf de poulet (*Gallus gallus*).

Dans le cadre de la présente invention, le terme « oeuf embryonné » désigne un oeuf d'oiseau fécondé dans lequel l'embryon peut se développer dans des conditions appropriées, en particulier dans un incubateur à une température de 37°C à 38°C. Dans ces conditions, le temps d'incubation nécessaire pour aboutir à l'éclosion de l'œuf est de 21 jours pour le poulet.

Les stades de développement renseignés ici sont définis en fonction du temps d'incubation post-fécondation des oeufs, en particulier du temps d'incubation dans les conditions appropriées telles que définies ci-dessus.

Par « greffe au niveau de la CAM », on entend désigner l'administration par apposition ou injection sur la CAM, que ce soit la CAM supérieure (*upper CAM*) ou inférieure (*lower CAM*).

Le modèle d'oeuf embryonné selon l'invention possède des cellules issues de deux organismes différents ou xénogreffes : les cellules de l'oiseau « hôte » ou « receveur » et les CTC greffées dans l'œuf qui sont issues d'un organisme humain ou animal d'une espèce différente de celle de l'oiseau « receveur ». De manière particulièrement préférée, les CTC greffées dans l'œuf embryonné d'oiseau sont des cellules humaines. Ces cellules CTC greffées vont ensuite se développer dans l'embryon en formant une ou plusieurs tumeurs solides et/ou en se déplaçant dans l'œuf.

Par définition, la « greffe au niveau de la CAM » a lieu une fois la CAM formée et à un stade équivalent à au moins 8 jours de développement chez le poulet, dans des conditions de croissances normales et standard. Si l'oiseau utilisé est le poulet, ce stade correspond à au moins 8 jours de développement. Le nombre de jours de développement pouvant varier d'une espèce à l'autre, la greffe peut intervenir à des jours de développement qui varient. Par exemple, un stade de développement d'au moins 8 jours chez le poulet correspond à un stade de développement d'au moins 6,5 jours chez la caille.

Par « échantillon de patient ou animal atteint de cancer », il faut comprendre tout échantillon issu d'un humain ou d'un animal atteint de cancer et qui contient des CTC. De préférence, ledit échantillon est choisi parmi le sang total ou des liquides biologiques susceptibles de contenir des CTC tels que liquide d'épanchement pleural, ascite et liquide céphalo-rachidien (LCR), et de préférence le sang total. Ainsi, les CTC greffées dans l'œuf embryonné peuvent provenir de tout type de cancer produisant des CTC dans le sang ou ce type de liquides, et en particulier des cancers métastatiques. Selon un mode de réalisation préféré de la présente invention, le patient ou l'animal à partir duquel sont isolées les CTC greffées dans l'œuf embryonné, est atteint d'au moins un cancer choisi parmi un cancer du poumon un cancer de la prostate, un cancer du sein, un cancer colorectal.

Avantageusement, le modèle d'oeuf embryonné selon l'invention est un oeuf de poulet dans lequel des CTC humaines ont été greffées au niveau de la CAM et à au moins 8 jours de développement.

Il est entendu que l'embryon greffé selon la présente invention n'a pas vocation à éclore et n'est par conséquent pas destiné à créer un organisme adulte. C'est en cela que l'embryon greffé selon la présente invention est un modèle destiné à recevoir les CTC le temps de leur amplification, n'allant pas jusqu'à l'éclosion, qui correspond à 21 jours de développement chez le poulet. En tout état de cause, l'embryon d'oiseau selon l'invention sera sacrifié, selon les règles d'éthique en vigueur, avant l'éclosion et après que les CTC greffées auront conduit au développement d'une ou plusieurs tumeurs dans l'œuf.

Un tel modèle permet à la fois d'amplifier des CTC, notamment par le développement de tumeurs à partir de ces CTC sur lesquelles pourront être testées l'activité anti-cancéreuse de différents agents, qui sont soit connus pour avoir une telle activité soit candidats pour déterminer s'ils possèdent une telle activité. L'efficacité comparée de plusieurs agents thérapeutiques pourra également être testée, afin de déterminer la thérapie la plus prometteuse pour traiter le patient dont sont issues les CTC greffées.

Selon un deuxième aspect, la présente invention est relative à un procédé de préparation du modèle d'oeuf embryonné d'oiseau décrit ci-dessus, permettant l'amplification de cellules tumorales circulantes (CTC) humaines ou animales isolées à partir d'un échantillon d'un patient ou d'un animal atteint de cancer, ledit procédé comprenant la greffe des CTC isolées à partir dudit échantillon au niveau de la membrane chorioallantoïque (CAM) d'un oeuf embryonné d'oiseau qui est à un stade de développement correspondant à la formation de la CAM et équivalent à au moins 8 jours de développement chez le poulet.

L'homme du métier saura déterminer le moment pour réaliser la greffe des CTC, en fonction de l'espèce d'oiseau utilisée, c'est-à-dire le nombre de jours d'incubation ou de développement minimum de l'œuf embryonné pour arriver à formation de la CAM et à un stade de développement équivalent à au moins 8 jours de développement chez le poulet. Par exemple, chez le poulet, la greffe pourra intervenir à partir de 8 jours de développement, et chez la caille à partir de 6,5 jours de développement.

Selon un mode de réalisation préféré, l'œuf embryonné a été, préalablement à la greffe, incubé jusqu'à un stade de développement correspondant à la formation de la CAM et équivalent à au moins 9 ou de manière encore plus préférée au moins 9,5 jours de développement chez le poulet.

Les incubations sont réalisées dans des conditions appropriées, c'est-à-dire des conditions qui permettent le développement normal de l'œuf embryonné, notamment à une température comprise entre 37°C et 39°C, et de préférence 38°C, voire 38,5°C.

La greffe des CTC peut être réalisée à n'importe quel endroit de la CAM, supérieure ou inférieure, de préférence au niveau de la CAM supérieure. Toute méthode bien connue de l'homme du métier pourra être utilisée pour cette greffe, et en particulier, il est possible d'utiliser la technique de greffe référencée par Crespo P. & Casar B, 2016.

Selon un mode de réalisation particulier, la quantité de CTC greffées va d'environ 5 CTC à environ 5000, de manière préférée d'environ 5 à environ 2500 et de manière encore préférée d'environ 5 à environ 1000 CTC.

Selon un mode de réalisation préféré, les CTC utilisées ont été congelées après isolement à partir de l'échantillon du patient ou de l'animal atteint de cancer et avant la greffe dans l'œuf embryonné.

Pour la greffe au niveau de la CAM, les CTC sont isolées à partir de l'échantillon du patient ou de l'animal atteint de cancer, par l'une quelconques des méthodes bien connues par l'homme du métier. Ainsi les CTC sont isolées en les purifiant des autres cellules présentes dans l'échantillon issu du patient ou de l'animal atteint de cancer, et en particulier en les séparant des cellules immunes présentes dans l'échantillon.

Les méthodes d'isolement des CTC peuvent reposer sur différents principes permettant de les séparer des autres constituants d'un échantillon, et par conséquence de procéder à un enrichissement en CTC. Un grand nombre de méthodes différentes ont notamment été décrites parZheyu Shen et al., 2017. Elles permettent d'aboutir à un enrichissement dit « négatif » lorsque l'objectif est de capturer les cellules non cibles et d'éluer les CTC, ou à un enrichissement dit « positif » lorsque l'objectif est de capturer les CTC et d'éluer les cellules non cibles de l'échantillon.

Parmi ces méthodes, on peut citer les méthodes de séparation par filtration, et en particulier par filtration verticale comme par exemple la technologie ISET (*Isolation by SizE of Tumor cell* ou Isolation par la taille des cellules tumorales) commercialisée par la société Rarecells SAS et notamment décrite par Han Wei Hou *et al.,* 2013.

Certaines méthodes peuvent faire appel à une étape de marquage, comme la méthode CellSearch (Kagan M, et al., 2002) qui repose sur une immuno-sélection des CTC utilisant des nanoparticules de ferrofluide avec des anticorps qui ciblent un marqueur spécifique d'adhésion des cellules épithéliales (EpCAM). Les CTC sont ainsi séparées magnétiquement de la majeure partie des autres cellules sanguines.

D'autres méthodes reposent sur l'utilisation de systèmes microfluidiques, qui reposent sur une séparation en fonction de différents paramètres comme la taille, la forme, la densité, la déformabilité des cellules, peuvent également être utilisées. Parmi celles-ci, on peut notamment citer la méthode VTX-1 telle que décrite par Sollier-Christen *et al.,* 2018, une méthode de microfluidique (CTC-chips) basée sur la capture des cellules tumorales exprimant la molécule EpCam à l'aide de microspots revêtus d'anticorps anti-EpCam, qui est notamment décrite par Nagrath S *et al.,* 2007, la technologie RosetteSep de la société Stemcells qui utilise un mélange d'anticorps (CD45, CD66b et glycophorine A) couplés à des billes magnétiques, ciblant les globules rouges et les globules blancs ou encore la méthode ClearCell Fx1 développée par la société Biolidics (anciennement ClearBridge), qui est en particulier décrite par Laget S *et al.,* 2017 et qui est particulièrement avantageuse.

Selon un mode de réalisation particulier, le procédé de préparation du modèle d'oeuf embryonné d'oiseau selon l'invention comprend en outre une étape d'incubation de l'œuf embryonné une fois greffé pendant au moins 12 heures, de préférence au moins 24 heures, de manière encore préférée au moins 48 heures après la greffe, avant d'être utilisé. De préférence l'œuf embryonné une fois greffé est incubé au maximum pendant 20 jours, et en particulier jusqu'à 18 jours, en référence aux stades de développement d'un embryon de poulet.

Selon un troisième aspect, la présente invention est relative à un procédé d'amplification de cellules tumorales circulantes (CTC) humaines ou animales isolées à partir d'un échantillon d'un patient ou d'un animal atteint de cancer, comprenant les étapes suivantes :
a) la greffe de CTC isolées à partir d'un échantillon d'un patient ou d'un animal atteint de cancer au niveau de la membrane chorioallantoïque (CAM) d'un oeuf embryonné d'oiseau qui est à un stade de développement correspondant à la formation de la CAM et équivalent à au moins 8 jours de développement chez le poulet, comme décrit ci-dessus,
b) le prélèvement des tumeurs qui se développent à partir des CTC qui se sont amplifiées, et éventuellement
c) la récupération des CTC à partir des tumeurs prélevées à l'étape b).

Les inventeurs ont démontré, de manière surprenante, que les CTC et en particulier des CTC d'origine humaine, peuvent être amplifiées dans un modèle d'oeuf embryonné d'oiseau, ce qui permet de les obtenir en des quantités suffisantes pour les étudier mais également de les utiliser pour conduire à l'identification de nouveaux traitements ou de sélectionner celui qui promet d'être le plus efficace de manière individualisée.

Selon un mode de réalisation préféré du procédé d'amplification de cellules tumorales circulantes (CTC) humaines ou animales décrit ci-dessus, il est possible de greffer à nouveau les CTC qui auront été amplifiées en effectuant par exemple plusieurs tours de greffe et de prélèvement (on parle de greffe secondaire pour deux tours de greffe), et ce afin d'améliorer davantage l'amplification des CTC. Ainsi, le procédé peut comprendre les étapes suivantes :
a) la greffe de CTC isolées à partir d'un échantillon d'un patient ou d'un animal atteint de cancer au niveau de la membrane chorioallantoïque (CAM) d'un oeuf embryonné d'oiseau qui est à un stade de développement correspondant à la formation de la CAM et équivalent à au moins 8 jours de développement chez le poulet,
b) le prélèvement des tumeurs qui se développent à partir des CTC qui se sont amplifiées,
   b1) la greffe des tumeurs prélevées à l'étape b), au niveau de la membrane chorioallantoïque (CAM) d'un deuxième oeuf embryonné d'oiseau préalablement incubé jusqu'à un stade de développement correspondant à la formation de la CAM et équivalent à au moins 8 jours de développement chez le poulet,
   b2) éventuellement l'incubation de ce deuxième oeuf embryonné ainsi greffé pendant au moins 12 heures,
   b3) le prélèvement des tumeurs qui se développent à partir des tumeurs greffées à l'étape b1), et éventuellement,
c) la récupération des CTC à partir des tumeurs prélevées à l'étape b3).

Selon un mode de réalisation particulier, le procédé d'amplification des cellules tumorales circulantes (CTC) humaines ou animales décrit ci-dessus comprend en outre une étape d'incubation de l'œuf embryonné une fois greffé pendant au moins 12 heures, de préférence au moins 24 heures, de manière encore préférée au moins 48 heures après la greffe, avant de procéder au prélèvement des tumeurs dérivées des CTC greffées. De préférence l'œuf embryonné une fois greffé est incubé au maximum pendant 20 jours, et en particulier jusqu'à 18 jours, en référence aux stades de développement d'un embryon de poulet.

La présente invention est ainsi également relative à un procédé de détermination de la sensibilité d'un patient ou d'un animal atteint de cancer à un ou plusieurs agent(s) thérapeutique(s), caractérisé en ce qu'il comprend :
- l'amplification de CTC isolées à partir d'un échantillon d'un patient ou d'un animal atteint de cancer dans un oeuf embryonné d'oiseau, comme décrit ci-dessus,
- la greffe des tumeurs ainsi prélevées, au niveau de la membrane chorioallantoïque (CAM) d'un nouvel oeuf embryonné d'oiseau préalablement incubé jusqu'à un stade de développement correspondant à la formation de la CAM et équivalent à au moins 8 jours de développement chez le poulet,
- l'administration du ou des agents thérapeutiques dans l'œuf embryonné au moins 12 heures après la greffe,
- l'étude de l'effet du ou des agents thérapeutiques ainsi administrés sur la tumorigenèse des tumeurs qui se sont développées dans ce nouvel oeuf embryonné greffé.

Par « agent thérapeutique » on entend désigner toutes molécules ou composés chimiques ou biologiques, nanostructure, procédés physique ou combinaison de ceux-ci ayant une action anti-tumorale, et en particulier potentiellement efficace pour traiter le type de cancer qui s'est développé à partir des cellules tumorales greffées dans l'œuf embryonné.

De tels composés peuvent être des molécules chimiques telles que des chimiothérapies, des composés biologiques telles que des anticorps, des cellules thérapeutiques telles que CART, des agents physiques tels que des irradiations, des agents intercalants comme par exemple l'Irinotecan, des inhibiteurs de voie de signalisation des tyrosines kinases comme par exemple le Sunitinib, les anti-hormonaux comme par exemple le Tamoxifène, les immunorégulateurs comme par exemple le Ketruda^{®}, les inhibiteurs de récepteurs membranaires comme par exemple le Trastuzumab, etc.

Dans le cadre de la présente invention, on utilise indifféremment, et avec la même signification, les termes tumeur et cancer, pour définir une prolifération de cellules malignes. Il en va de même avec l'utilisation des termes anti-tumoral et anticancéreux.

En particulier, puisque les CTC greffées sont isolées à partir d'un échantillon de patient ou d'animal atteint de cancer, le fait de tester plusieurs agents thérapeutiques permet de pouvoir sélectionner celui qui est le plus prometteuse pour le traitement de la tumeur chez ce patient. C'est ainsi que selon un mode de réalisation préféré de la présente invention, l'œuf embryonné d'oiseau greffé avec des CTC est utilisé pour déterminer l'agent qui présente la meilleure activité anti-cancéreuse parmi les différents testés.

L'oeuf embryonné d'oiseau greffé avec les CTC peut également être utilisé selon la présente invention pour tester l'efficacité anti-cancéreuse de combinaisons d'agents thérapeutiques par rapport à l'effet obtenu avec chacun des agents testés indépendamment.

Dans le cadre de l'utilisation de cet oeuf embryonné, il est aussi possible de déterminer, voire quantifier, la toxicité du ou des agents thérapeutiques testées, à la fois sur les tumeurs qui se sont développées à partir des CTC greffées et sur l'embryon dans son ensemble. Par conséquent, un autre objet de la présente invention concerne l'utilisation d'un oeuf embryonné d'oiseau greffé avec des CTC isolées à partir d'un échantillon d'un patient ou d'un animal atteint de cancer pour quantifier la toxicité d'un ou de plusieurs agent(s) thérapeutique(s) sur la tumeur et/ou sur l'embryon dans son ensemble.

L'étape d'administration du ou des agents thérapeutiques dans l'œuf embryonné peut être réalisée de différentes manières par des techniques bien connues de l'homme du métier. L'administration peut notamment être réalisée par apposition ou injection au niveau de la CAM, par injection intra-tumorale, par injection dans les structures embryonnaires ou extra-embryonnaires de l'œuf.

L'administration du ou des agents thérapeutiques est réalisée au moins 12h après la greffe des CTC, de préférence au moins 24h ou de manière encore préférée au moins 48h après la greffe, c'est-à-dire 1 à 2 jours après la greffe. L'agent ou les agents thérapeutiques peuvent également être administrés selon différents schémas en termes de durée, mais également de nombre d'administrations, comme par exemple tous les deux jours, ou tous les jours, ou deux fois par jour, ou une injection unique, et ce jusqu'au dernier jour d'incubation de l'œuf. Ces choix seront déterminés en fonction de l'agent administré.

Selon un mode de réalisation préféré, le procédé de détermination de la sensibilité d'un patient ou d'un animal atteint de cancer à un ou plusieurs agent(s) thérapeutique(s) selon l'invention, comprend en outre l'incubation de l'œuf embryonné une fois greffé pendant au moins 1 heure, après administration du ou des agents thérapeutiques dans l'œuf embryonné greffé avant d'étudier l'effet sur la tumorigenèse. Avantageusement, l'incubation est réalisée pendant au moins 4 jours et au maximum 12 jours après administration, pour correspondre à un stade de développement de l'embryon de 21 jours maximum, et avantageusement de 18 jours de développement. Selon un mode de réalisation particulier, le procédé de détermination de la sensibilité d'un patient ou d'un animal atteint de cancer à un ou plusieurs agent(s) thérapeutique(s) selon l'invention comprend en outre le prélèvement des tumeurs qui se développent à partir des CTC greffées au terme de l'incubation dudit oeuf embryonné après administration du ou des agents thérapeutiques qui ont été administrées, et notamment par microdissection. L'étude de l'effet du ou des agents thérapeutiques ainsi administrée(s) sur la tumorigenèse peut revêtir plusieurs approches complémentaires, en particulier après prélèvement des tumeurs qui se sont développées dans l'œuf embryonné greffé. Elle peut notamment comprendre l'analyse de paramètres tels que la croissance tumorale, l'invasion métastatique, l'angiogenèse, la néo-angiogenèse, l'inflammation et/ou l'infiltration immunitaire tumorale, la toxicité sur la tumeur et/ou sur l'embryon dans son ensemble.

Les tumeurs peuvent ainsi être soumises à des analyses pour mesurer et/ou analyser ces différents paramètres, telles que le poids et/ou le volume tumoral pour étudier la croissance tumorale, l'expression de différents marqueurs spécifiques pour étudier l'invasion métastatique tels que l'amplification de séquence Alu par PCR quantitative pour les métastase humaines, le nombre de vaisseaux à la tumeur pour l'angiogenèse et la néo-angiogenèse, la quantification des interleukines pour l'inflammation et/ou la quantification, notamment par rtQPCR, de marqueurs tels que CD3, CD8, CD4, CD45 et CD56 pour apprécier l'infiltration immunitaire tumorale, le poids, et des analyses histologiques pour évaluer la toxicité sur la tumeur.

L'étude de l'invasion métastatique peut être réalisée sur la CAM inférieure, facilement accessible, mais elle peut aussi être réalisée dans n'importe quel organe cible au sein de l'embryon, notamment en fonction du type de cancer et des données connues sur le phénomène de métastases associé.

L'inflammation et/ou l'infiltration immunitaire tumorale peut notamment être étudiée par l'analyse de l'expression de différents marqueurs, tels que CD3 (marqueur membranaire des lymphocytes T), CD4 (marqueur membranaire des lymphocytes T régulateurs, monocytes et macrophages), CD8 (marqueur des lymphocytes T cytotoxiques), CD45 (marqueur membranaire des leucocytes), CD56 (marqueur de cellules NK), etc. Des couples d'oligonucléotides spécifiques de ces marqueurs pourront être développés, afin d'éviter le croisement inter-espèces.

Par extension, il est également possible de suivre l'inflammation et l'infiltration de cellules du système immunitaire dans les sites de métastases.

L'analyse combinée de tous ces facteurs, bien connus de l'homme du métier, permet de déterminer la sensibilité du patient ou de l'animal atteint de cancer dont sont issues les CTC greffées à l'agent ou aux agents thérapeutique(s) administrés dans l'embryon. Ces paramètres font notamment partie intégrante de l'arbre décisionnel utilisé par les cliniciens pour décider de la prise en charge thérapeutique à adopter chez les patients atteints de cancers.

Dans le cadre de tous les procédés selon l'invention qui comprennent l'effet du ou des agents thérapeutiques sur la tumorigenèse, ceci est préférentiellement apprécié par comparaison de ces paramètres après administration du ou des agents thérapeutiques dans l'œuf embryonné une fois greffé par rapport à ceux déterminés dans un autre oeuf embryonné du même oiseau préalablement greffé selon le même procédé avec les mêmes CTC mais dans lequel aucun agent thérapeutique n'a été administrée. De même, lorsque l'effet de plusieurs agents thérapeutiques est étudié, l'effet sera préférentiellement apprécié par comparaison des paramètres après administration de la combinaison d'agents thérapeutiques dans l'œuf embryonné une fois greffé par rapport à ceux déterminés dans un ou plusieurs autre(s) oeuf(s) embryonné(s) du même oiseau préalablement greffé(s) selon le même procédé avec les mêmes CTC mais dans le(s)quel(s) chacun des agents thérapeutiques a été administré individuellement.

Selon un autre aspect, l'invention se rapporte également à un procédé de suivi d'un patient ou d'un animal atteint de cancer, comprenant :
- la préparation d'un premier oeuf embryonné d'oiseau comme décrit ci-dessus avec des CTC isolées à partir d'un échantillon dudit patient ou animal à un instant T1, et l'étude de la tumorigenèse des tumeurs qui se développent dans ce premier oeuf embryonné,
- la préparation d'un second oeuf embryonné d'oiseau comme décrit ci-dessus avec des CTC isolées à partir d'un échantillon du même patient ou animal à un instant T2, et l'étude de la tumorigenèse des tumeurs qui se développent dans ce second oeuf embryonné,
- la comparaison de la tumorigenèse des tumeurs qui se sont développées dans le premier et dans le second oeuf embryonné.

La présente invention a également pour objet un procédé de criblage d'agents thérapeutiques destinés au traitement du cancer *in vivo,* comprenant :
- la greffe de cellules tumorales circulantes isolées à partir d'un échantillon de patient ou d'un animal atteint de cancer, au niveau de la CAM d'un oeuf embryonné d'oiseau préalablement incubé jusqu'à un stade de développement correspond à la formation de la CAM et équivalent à au moins 8 jours de développement chez le poulet,
- l'administration d'un ou de plusieurs agents candidats dans l'œuf embryonné, au moins 12 heures après la greffe,

- l'étude de l'effet du ou des agents thérapeutiques ainsi administrés sur la tumorigenèse des tumeurs qui se sont développées dans l'œuf embryonné greffé. Par « agent thérapeutique candidat », on entend un agent thérapeutique chimique ou biologique tel que défini ci-dessus et susceptible d'avoir une activité anti-cancéreuse, et en particulier potentiellement efficace pour traiter le type de cancer qui s'est développé à partir des cellules tumorales greffées dans l'œuf embryonné. Le procédé de criblage selon la présente invention permet de déterminer si un agent thérapeutique candidat présente ou non une activité anti-cancéreuse, et s'il possède une activité anti-métastase.

Toutes les préférences et précisions mentionnées ci-dessus dans le cadre des différents procédés s'appliquent également aux procédés de suivi et de criblage selon la présente invention.

### EXEMPLES

L'invention est illustrée ci-après par l'utilisation de l'œuf de poule embryonné pour amplifier des CTC humaines.

### Préparation des CTC

Les CTC greffées dans les oeufs embryonnés sont isolées par filtration basée sur la taille des cellules selon la méthode ISET (Han Wei Hou *et al.,* 2013) ou par système microfluidique selon la méthode ClearCell (Laget S *et al.,* 2017) à partir d'échantillons de sang de patients atteints d'un cancer du poumon, cancer du sein, ou cancer de la prostate en phase métastatique. Les CTC ainsi obtenues sont stockées congelées, et décongelées juste avant la greffe.

### Induction de tumeurs

Des oeufs de poules Leghorn blanche fécondés ont été incubés en position couchée pendant 9 à 10 jours à 37,5°C avec une humidité relative de 40%. Après cette période d'incubation (E9 ou E10), les oeufs ont été préparés pour recevoir la greffe. Ils sont ouverts en préservant l'intégrité de la CAM, qui est abaissée en faisant un petit trou à travers la coquille. Puis une fenêtre d'environ 1 cm² est découpée au-dessus de la CAM. Les CTC (5 à 2500 cellules) sont greffées par apposition sur la CAM puis les oeufs sont à nouveau incubés à 37,5°C et 40% d'humidité. Après la greffe, les tumeurs *in ovo* se développent sur la CAM supérieure. Les oeufs sont observés toutes les 48h minimum pour le suivi du développement.

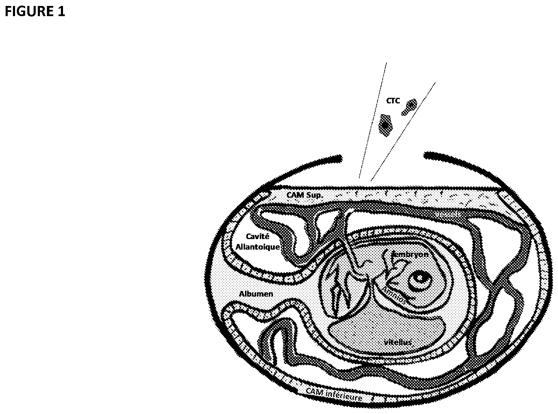

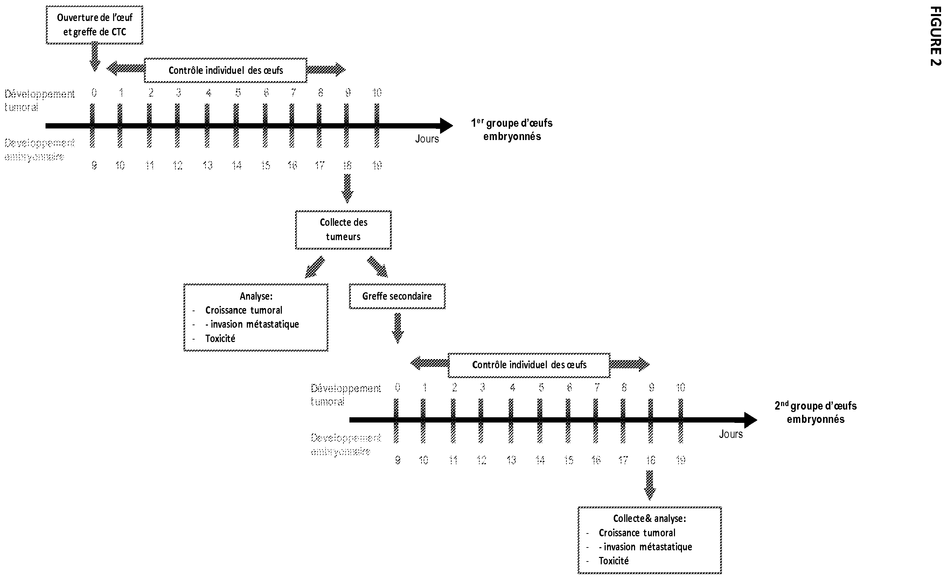

Un schéma d'oeuf embryonné avec le site de greffe de CTC sur la CAM supérieure est représenté sur la Figure 1. Un exemple de schéma expérimental est représenté sur la Figure 2.

A jour 18 du développement (E18), la partie supérieure de la CAM est retirée, lavée dans du tampon phosphate salin puis transférée directement dans du paraformaldéhyde (PFA) pour une fixation pendant 48 heures. Les tumeurs sont ensuite soigneusement extraites du tissu CAM normal.

Les tumeurs prélevées sont ensuite analysées, et notamment :
- pesées pour étudier la croissance tumorale (Figure 2, premier tour) ;
- utilisées pour des analyses histologiques ou génétiques (métastases par exemple) (Figure 2, premier tour) ;
- utilisées pour une greffe secondaire dans un nouveau lot d'oeufs embryonnés à 9 jours de développement pour amplification (Figure 2, deuxième tour). Cette étape peut être répétée autant que nécessaire (avec contrôle du phénotype régulièrement).

### Greffes secondaires

Après 9 jours de croissance des CTC greffées, les tumeurs qui se sont développées sont collectées et préparées pour la réalisation d'une greffe secondaire. La tumeur est coupée en petits morceaux et chaque pièce est greffée dans la CAM d'un nouvel oeuf afin d'obtenir une forte amplification des CTC.

### Analyse des métastases

Une analyse des métastases peut être réalisée soit sur un morceau de la CAM inférieure (à l'opposé du site de greffe) soit sur des tissus embryonnaires collectés en même temps que la tumeur et stockés de manière idoine. Après extraction d'ADN génomique total la mise en évidence des cellules humaines dans ces échantillons peut être réalisée par qPCR à l'aide amorces spécifiques pour les séquences Alu humaines (séquences en multi copies bien conservées chez l'homme).

### Résultats

Le schéma expérimental décrit ci-dessus a été appliqué avec des CTC isolées à partir du sang de patients atteints du cancer du poumon (#DA106, #DA107, #PA108, #CB110, #PC111, #VM109, #SH103, #CM105), des CTC isolées à partir de sang de patientes atteintes du cancer du sein (#PS234, #SS226, #UR227, #LP229), de CTC isolées à partir de sang de patients atteints du cancer de la prostate (#PT319, #BG320).

Après 9 jours sur CAM (E18), tous les échantillons de patients CTC ont été greffés avec succès sur la CAM et ont présenté un développement de tumeur. Les images *in ovo* de la croissance des tumeurs après un premier tour d'amplification sont regroupées dans la Figure 3.

Des analyses histologiques par coloration Hématoxyline/Eosine ont été réalisées sur les tumeurs prélevées au premier tour d'amplification et dérivées des CTC isolées à partir de sang de la patiente atteinte du cancer du sein (#PS234), puis greffées sur la CAM supérieure.

Les résultats sont regroupés dans la Figure 4, qui montre la présence d'une prolifération tumorale maligne.

Enfin, des analyses de mutations génétiques ont été réalisées par du séquençage à haut débit selon la méthode « Next Génération Sequencing » (NGS) référencée en 2017 dans la revue de Yohe S & Thyagarajan B. avec les marqueurs tumoraux humains TP53 (Mogi A & Kuwano H 2011) et KRAS (Gou LY et al., 2015) sur de l'ADN génomique extrait des tumeurs prélevées au premier tour d'amplification et dérivées de CTC isolées de sang de patients atteints du cancer du poumon. L'extraction d'ADN a été réalisée selon le procédé du kit GeneJET FFPE DNA Purification Kit commercialisé par ThermoFisher.

Les résultats sont regroupés dans la Figure 5, qui montre la présence de mutations des marqueurs tumoraux humains TP53 (Figure 5A) et KRAS (Figure 5B) dans deux tumeurs distinctes issues d'une première greffe de CTC de patients atteints de cancer du poumon (#SH103 et #CM105).

Les tumeurs issues d'une première greffe de CTC isolées à partir du sang d'un patient atteint d'un cancer du poumon ont été prélevées à E18, découpées puis à nouveau greffées sur la CAM d'un deuxième groupe d'oeufs embryonnés. Les images *in ovo* de croissance de tumeurs prélevées à E18 après un second tour d'amplification sont regroupées dans la Figure 6 (A et B correspondent à des images de tumeurs *in ovo* sur CAM issues de deux greffes secondaires distinctes).

Les tumeurs issues d'une première greffe de CTC isolées à partir du sang d'un patient atteint d'un cancer de la prostate (méthode microfluidique ClearCell Fx1) ont été prélevées à E18, découpées puis à nouveau greffées sur la CAM d'un deuxième groupe d'oeufs embryonnés. Les images *in ovo* de croissance de tumeurs sont regroupées dans la Figure 7. L'image A représente la tumeur obtenue *in ovo* à partir des CTC suite à la greffe primaire. Les deux sites de prise tumorale sont montrés par les flèches. L'image B correspond à celle d'une tumeur *in ovo* sur CAM issue d'une greffe secondaire, réalisée à partir de la tumeur montrée en A. L'image C correspond à l'analyse histologique d'une coupe de la tumeur représentée en B, montrant d'une part le tissu de la CAM, et d'autre part le tissu tumoral.

### Conclusions

Les résultats exposés ci-dessus montrent que l'utilisation d'un modèle d'oeuf embryonné aviaire permet l'amplification *in ovo* de CTC isolées à partir d'échantillons de patients, de manière plus rapide et moins coûteuse par rapport au modèle murin de l'art antérieur. La présente invention permet donc de constituer rapidement des bio-banques de CTC, qui peuvent ensuite être utilisées pour des tests d'efficacité de thérapies anti-cancéreuses, de suivi et de criblage de nouveaux agents susceptibles de pouvoir donner lieu à des traitements efficaces et personnalisés.

### REFERENCES

Kowalik A et al. Current approaches for avoiding the limitations of circulating tumor cells détection methods and treatment of patients with solid tumors. Translational Res. 2017. A review article, Vol 185 : 58-84;
Drapkin B. J. et al. Genomic and Functional Fidelity of small cell long cancer Patient-Derived Xenografts. Cancer Discov. 2018 May;8(5):600-615.
Pantel K, Alix-Panabières C. Functional Studies on Viable Circulating Tumor Cells. Clin Chem. 2016 Feb;62(2):328-34.
Giuliano M, Herrera S, Christiny P, Shaw C, Creighton CJ, Mitchell T, Bhat R, Zhang X, Mao S, Dobrolecki LE, Al-rawi A, Chen F, Veneziani BM, Zhang XH, Hilsenbeck SG, Contreras A, Gutierrez C, Jeselsohn RM, Rimawi MF, Osborne CK, Lewis MT, Schifif R, Trivedi MV. Circulating and disseminated tumor cells from breast cancer patient-derived xenograft-bearing mice as a novel model to study metastasis. Breast Cancer Res. 2015 Jan 9;17:3.
Williams ES, Rodriquez-Bravo V, Chippada-Venkata U, De la Iglesia-Vicente J, Gong Y, Galsky M, Oh W, Cordon-Cardo C, Domingo-Domenech J. Génération of Prostate Cancer Patient Derived Xenograft Models from Circulating Tumor Cells. J Vis Exp. 2015 Oct 20;(105):53182.
Torphy RJ, Tignanelli CJ, Kamande JW, Moffitt RA, Herrera Loeza SG, Soper SA, Yeh JJ. Circulating tumor cells as a biomarker of response to treatment in patient-derived xenograft mouse models of pancreatic adenocarcinoma. PLoS One. 2014 Feb 19;9(2)
Rossi E, Rugge M, Facchinetti A, Pizzi M, Nardo G, Barbieri V, Manicone M, De Faveri S, Chiar a Scaini M, Basso U, Amadori A, Zamarchi R. Retaining the long-survive capacity of Circulating Tumor Cells (CTCs) followed by xeno-transplantation: not only from metastatic cancer of the breast but also of prostate cancer patients. Oncoscience. 2013 Dec 31;1(1):49-56
Hodgkinson, C. L., Morrow, C. J., Li, Y., Metcalf, R. L., Rothwell, D. G., Trapani, F., Polanski, R., Burt, D. J., Simpson, K. L., Morris, K., Pepper, S. D., Nonaka, D., Greystoke, A., Kelly, P., Bola, B., Krebs, M. G., Antonello, J., Ayub, M., Faulkner, S., Priest, L., Carter, L., Tate, C., Miller, C. J., Blackhall, F., Brady, G., and Dive, C. (2014). Tumorigenicity and genetic profiling of circulating tumor cells in small-cell lung cancer. Nat Med 20, 897-903.
Crespo P. & Casar B.; Bio-protocol, Vol. 6, Iss 20, Oct 20, 2016; Chick Embryo Chorioallantoic Membrane as an in vivo Model to Study Metastasis
Han Wei Hou et al. Scientific Reports 3, Article number: 1259 (2013)
Laget S et al.. PloS one, 2017 Jan 6;12(1):e0169427.
Yohe S et Thyagarajan B. Arch Pathol Lab Med. 2017 Nov;141(11):1544-1557. Review of Clinical Next-Generation Sequencing
Mogi A and Kuwano H ; J Biomed Biotechnol. 2011;2011:583929. TP53 mutations in nonsmall cell lung cancer.
Gou LY et al. ; Cancer. 2015 Sep 1;121 Suppl 17:3069-79. Différences in driver genes between smoking-related and non-smoking-related lung cancer in the Chinese population.
Zheyu Shen et al., Chem Soc Rev, 2017 Apr 18;46(8): 2038-2056. Current Détection Technologies of Circulating Tumor Cells.
Sollier-Christen et al., Journal of Quantitative Cell Science, Cytometry Part A, 2018, 93A : 1240-1245.
Kagan M, Howard D, Bendele T, et al A sample préparation and analysis system for identification of circulating tumor cells. J Clin Ligand Assay 2002;25:104-10.
Nagrath S1, Sequist LV, Maheswaran S, Bell DW, Irimia D, Ulkus L, Smith MR, Kwak EL, Digumarthy S, Muzikansky A, Ryan P, Balis UJ, Tompkins RG, Haber DA, Toner M ; Nature. 2007 Dec 20;450(7173):1235-9.

## Revendications

1. Modèle d'oeuf embryonné d'oiseau comprenant des cellules tumorales circulantes (CTC) humaines ou animales isolées à partir d'un échantillon d'un patient ou d'un animal atteint de cancer, lesdites CTC étant greffées au niveau de la membrane chorioallantoïque (CAM) d'un oeuf embryonné étant à un stade de développement qui correspond à la formation de la CAM et équivalent à au moins 8 jours de développement chez le poulet au moment de la greffe.

2. Modèle selon la revendication 1, **caractérisé en ce que** l'œuf embryonné est à un stade de développement d'au plus 20 jours.

3. Procédé de préparation d'un modèle d'oeuf embryonné d'oiseau selon l'une quelconque des revendications 1 ou 2 permettant l'amplification de cellules tumorales circulantes (CTC) humaines ou animales isolées à partir d'un échantillon d'un patient ou d'un animal atteint de cancer, ledit procédé comprenant les étapes suivantes :
- la greffe des CTC isolées à partir dudit échantillon au niveau de la membrane chorioallantoïque (CAM) d'un oeuf embryonné d'oiseau qui est à un stade de développement correspondant à la formation de la CAM et équivalent à au moins 8 jours de développement chez le poulet,
- éventuellement l'incubation de l'œuf embryonné ainsi greffé pendant au moins 12 heures.

4. Procédé d'amplification de cellules tumorales circulantes (CTC) humaines ou animales isolées à partir d'un échantillon d'un patient ou d'un animal atteint de cancer, comprenant les étapes suivantes :
a) la préparation d'un modèle d'oeuf embryonné à l'aide du procédé selon la revendication 3,
b) le prélèvement des tumeurs qui se développent à partir des CTC qui se sont amplifiées,
c) éventuellement la récupération des CTC à partir des tumeurs prélevées à l'étape b).

5. Procédé d'amplification de cellules tumorales circulantes (CTC) humaines ou animales selon la revendication 4, **caractérisé en ce qu'**il comprend les étapes suivantes :
a) la préparation d'un premier modèle d'oeuf embryonné à l'aide du procédé selon la revendication 3,
b) le prélèvement des tumeurs qui se développent à partir des CTC qui se sont amplifiées,
b1) la greffe des tumeurs prélevées à l'étape b), au niveau de la membrane chorioallantoïque (CAM) d'un deuxième oeuf embryonné d'oiseau préalablement incubé jusqu'à un stade de développement correspondant à la formation de la CAM et équivalent à au moins 8 jours de développement chez le poulet,
b2) éventuellement l'incubation de ce deuxième oeuf embryonné ainsi greffé pendant au moins 12 heures,
b3) le prélèvement des tumeurs qui se développent à partir des tumeurs greffées à l'étape b1), et éventuellement,
c) la récupération des CTC à partir des tumeurs prélevées à l'étape b3).

6. Procédé de détermination de la sensibilité d'un patient ou d'un animal atteint de cancer à un ou plusieurs agent(s) thérapeutique(s), **caractérisé en ce qu'**il comprend :
- l'amplification de cellules tumorales circulantes (CTC) isolées à partir d'un échantillon dudit patient ou animal atteint de cancer selon le procédé tel que défini à l'une quelconque des revendications 4 à 5,
- la greffe des tumeurs ainsi prélevées, au niveau de la membrane chorioallantoïque (CAM) d'un nouvel oeuf embryonné d'oiseau préalablement incubé jusqu'à un stade de développement correspondant à la formation de la CAM et équivalent à au moins 8 jours de développement chez le poulet,
- l'administration du ou des agents thérapeutiques dans l'œuf embryonné au moins 12 heures après la greffe,
- l'étude de l'effet du ou des agents thérapeutiques ainsi administrés sur la tumorigenèse des tumeurs prélevées qui se sont développées dans ce nouvel oeuf embryonné greffé.

7. Procédé de détermination de la sensibilité d'un patient ou d'un animal atteint de cancer à une ou plusieurs agent(s) thérapeutique(s) selon la revendication 6, **caractérisé en ce que** l'étude de la tumorigenèse comprend l'appréciation ou la mesure de différents paramètres tels que la croissance tumorale, l'invasion métastatique, l'angiogenèse, la néo-angiogenèse, l'inflammation et/ou l'infiltration immunitaire tumorale, la toxicité sur la tumeur et/ou sur l'embryon dans son ensemble.

8. Procédé de suivi d'un patient ou d'un animal atteint de cancer, comprenant :
- la préparation d'un premier oeuf embryonné d'oiseau selon la revendication 3 avec des CTC isolées à partir d'un échantillon dudit patient ou animal à un instant T1, et l'étude de la tumorigenèse des tumeurs qui se développent dans ce premier oeuf embryonné,
- la préparation d'un second oeuf embryonné d'oiseau selon la revendication 3 avec des CTC isolées à partir d'un échantillon du même patient ou animal à un instant T2, et l'étude de la tumorigenèse des tumeurs qui se développent dans ce second oeuf embryonné,
- la comparaison de la tumorigenèse des tumeurs qui se sont développées dans le premier et dans le second oeuf embryonné.

9. Procédé de criblage d'agents thérapeutiques destinés au traitement du cancer *in vivo,* comprenant :
- la préparation d'un modèle d'oeuf embryonné à l'aide du procédé selon la revendication 3,
- l'administration d'un ou de plusieurs agents candidats dans l'œuf embryonné au moins 12 heures après la greffe,
- l'étude de l'effet du ou des agents thérapeutiques ainsi administrés sur la tumorigenèse des tumeurs qui se sont développées dans l'œuf embryonné greffé.

## Patentansprüche

1. Vogelembryoneneimodell, das menschliche oder tierische zirkulierende Tumorzellen (CTC) umfasst, die aus einer Probe eines Patienten oder eines Tieres isoliert wurden, der/das unter Krebs leidet, wobei die CTC im Bereich der Chorioallantoismembran (CAM) eines Embryoneneis in einem Entwicklungsstadium transplantiert werden, das der Bildung der CAM entspricht und mindestens 8 Tagen Entwicklung beim Huhn zum Zeitpunkt der Transplantation entspricht.

2. Modell nach Anspruch 1, **dadurch gekennzeichnet, dass** das Embryonenei in einem Entwicklungsstadium von höchstens 20 Tagen ist.

3. Verfahren zur Herstellung eines Vogelembryoneneimodells nach einem der Ansprüche 1 oder 2, das die Amplifizierung menschlicher oder tierischer zirkulierender Tumorzellen (CTC) umfasst, die aus einer Probe eines Patienten oder eines Tieres isoliert wurden, der/das unter Krebs leidet, wobei das Verfahren die folgenden Schritte umfasst:
- das Transplantieren der aus der Probe isolierten CTC im Bereich der Chorioallantoismembran (CAM) eines Vogelembryoneneis, das in einem Entwicklungsstadium ist, das der Bildung der CAM entspricht und mindestens 8 Tagen Entwicklung beim Huhn entspricht,
- eventuell das Inkubieren des derart transplantierten Embryoneneis für mindestens 12 Stunden.

4. Verfahren zur Amplifizierung von menschlichen oder tierischen zirkulierenden Tumorzellen (CTC), die aus einer Probe eines Patienten oder eines Tieres isoliert wurden, der/das unter Krebs leidet, umfassend die folgenden Schritte:
a) das Herstellen eines Embryoneneimodells mit Hilfe des Verfahrens nach Anspruch 3,
b) das Entnehmen der Tumoren, die sich aus den amplifizierten CTC entwickeln,
c) eventuell das Rückgewinnen der CTC aus den in Schritt b) entnommenen Tumoren.

5. Verfahren zur Amplifizierung von menschlichen oder tierischen zirkulierenden Tumorzellen (CTC) nach Anspruch 4, **dadurch gekennzeichnet, dass** es die folgenden Schritte umfasst:
a) das Herstellen eines ersten Embryoneneimodells mit Hilfe des Verfahrens nach Anspruch 3,
b) das Entnehmen der Tumoren, die sich aus den amplifizierten CTC entwickeln,
b1) das Transplantieren der in Schritt b) entnommenen Tumoren im Bereich der Chorioallantoismembran (CAM) eines zweiten Vogelembryoneneis, das zuvor bis zu einem Entwicklungsstadium inkubiert wurde, das der Bildung der CAM entspricht und mindestens 8 Tagen Entwicklung beim Huhn entspricht,
b2) eventuell das Inkubieren dieses zweiten derart transplantierten Embryoneneis für mindestens 12 Stunden,
b3) das Entnehmen der Tumoren, die sich aus den in Schritt b1) transplantierten Tumoren entwickeln, und eventuell
c) das Rückgewinnen der CTC aus den in Schritt b3) entnommenen Tumoren.

6. Verfahren zur Bestimmung der Sensibilität eines Patienten oder eines Tieres, der/das an Krebs leidet, für ein oder mehrere therapeutische(s) Mittel, **dadurch gekennzeichnet, dass** es umfasst:
- das Amplifizieren zirkulierender Tumorzellen (CTC), die aus einer Probe des Patienten oder des Tieres isoliert wurden, der/das unter Krebs leidet, nach dem Verfahren nach einem der Ansprüche 4 bis 5,
- das Transplantieren der derart entnommenen Tumoren im Bereich der Chorioallantoismembran (CAM) eines neuen Vogelembryoneneis, das zuvor bis zu einem Entwicklungsstadium inkubiert wurde, das der Bildung der CAM entspricht und mindestens 8 Tagen Entwicklung beim Huhn entspricht,
- das Verabreichen des oder der therapeutischen Mittel in das Embryonenei mindestens 12 Stunden nach der Transplantation,
- das Untersuchen der Wirkung des oder der derart verabreichten therapeutischen Mittel auf die Tumorentwicklung der entnommenen Tumoren, die sich in diesem neuen transplantierten Embryonenei entwickelt haben.

7. Verfahren zur Bestimmung der Sensibilität eines Patienten oder eines Tieres, der/das an Krebs leidet, für ein oder mehrere therapeutische(s) Mittel nach Anspruch 6, **dadurch gekennzeichnet, dass** die Untersuchung der Tumorentwicklung die Beurteilung oder die Messung verschiedener Parameter wie Tumorwachstum, Metastaseninvasion, Angiogenese, Neo-Angiogenese, Entzündung und/oder tumorale Immuninfiltration, Toxizität auf den Tumor und/oder auf den Embryo insgesamt umfasst.

8. Verfahren zur Beobachtung eines Patienten oder eines Tieres, das unter Krebs leidet, umfassend:
- das Herstellen eines ersten Vogelembryoneneis nach Anspruch 3 mit CTC, die aus einer Probe des Patienten oder Tieres isoliert wurden zu einem Zeitpunkt T1, und das Untersuchen der Tumorentwicklung der Tumoren, die sich in diesem ersten Embryonenei entwickeln,
- das Herstellen eines zweiten Vogelembryoneneis nach Anspruch 3 mit CTC, die aus einer Probe desselben Patienten oder Tieres isoliert wurden zu einem Zeitpunkt T2, und das Untersuchen der Tumorentwicklung der Tumoren, die sich in diesem zweiten Embryonenei entwickeln,
- das Vergleichen der Entwicklung der Tumoren, die sich im ersten und im zweiten Embryonenei entwickelt haben.

9. Verfahren zum Screening therapeutischer Mittel, die zur Behandlung des Krebses *in vivo* bestimmt sind, umfassend:
- das Herstellen eines Embryoneneimodells mit Hilfe des Verfahrens nach Anspruch 3,
- das Verabreichen von einem oder mehreren Mitteln in das Embryonenei mindestens 12 Stunden nach dem Transplantieren,
- das Untersuchen der Wirkung des oder der derart verabreichten therapeutischen Mittel auf das Wachstum der Tumoren, die sich im transplantierten Embryonenei entwickelt haben.

## Claims

1. Embryonated bird's egg model comprising human or animal circulating tumour cells (CTC) isolated from a sample of a patient or an animal suffering from cancer, said CTC being grafted at the level of the chorioallantoic membrane (CAM) of an embryonated egg being at a development stage which corresponds to the formation of the CAM and equivalent to at least 8 days of development in chickens at the moment of the graft.

2. Model according to claim 1, **characterised in that** the embryonated egg is at a development stage of at the most 20 days.

3. Method for preparing an embryonated bird's egg model according to any one of claims 1 or 2 enabling the amplification of human or animal circulating tumour cells (CTC) isolated from a sample of a patient or an animal suffering from cancer, said method comprising the following steps:
- grafting CTC isolated from said sample at the level of the chorioallantoic membrane (CAM) of an embryonated bird's egg which is at a development stage corresponding to the formation of the CAM and equivalent to at least 8 days of development in chickens,
- optionally incubation of the embryonated egg thereby grafted for at least 12 hours.

4. Method for amplifying human or animal circulating tumour cells (CTC) isolated from a sample of a patient or an animal suffering from cancer, comprising the following steps:
a) preparation of an embryonated egg model using the method according to claim 3,
b) collection of tumours that develop from CTC that are amplified,
c) optionally, recovery of CTC from the tumours collected at step b).

5. Method for amplifying human or animal circulating tumour cells (CTC) according to claim 4, **characterised in that** it comprises the following steps:
a) preparation of a first embryonated egg model using the method according to claim 3,
b) collection of tumours that develop from CTC that are amplified,
b1) grafting the tumours collected at step b) at the level of the chorioallantoic membrane (CAM) of a second embryonated bird's egg incubated beforehand up to a development stage corresponding to the formation of the CAM and equivalent to at least 8 days of development in chickens,
b2) optionally, incubation of this second embryonated egg thereby grafted for at least 12 hours,
b3) collection of tumours that develop from the tumours grafted at step b1), and optionally,
c) recovery of CTC from the tumours collected at step b3).

6. Method for determining the sensitivity of a patient or an animal suffering from cancer to one or more therapeutic agent(s), **characterised in that** it comprises:
- amplification of circulating tumour cells (CTC) isolated from a sample of said patient or animal suffering from cancer according to the method such as defined in any one of claims 4 to 5,
- grafting the tumours thereby collected at the level of the chorioallantoic membrane (CAM) of a new embryonated bird's egg incubated beforehand up to a development stage corresponding to the formation of the CAM and equivalent to at least 8 days of development in chickens,
- administration of the therapeutic agent(s) in the embryonated egg at least 12 hours after the graft,
- study of the effect of the therapeutic agent(s) thereby administered on tumorigenesis of the collected tumours that have developed in this new grafted embryonated egg.

7. Method for determining the sensitivity of a patient or an animal suffering from cancer to one or more therapeutic agent(s) according to claim 6, **characterised in that** the study of tumorigenesis comprises the assessment or the measurement of different parameters such as tumour growth, metastatic invasion, angiogenesis, neo-angiogenesis, inflammation and/or tumour immune infiltration, toxicity on the tumour and/or on the embryo as a whole.

8. Method for monitoring a patient or an animal suffering from cancer, comprising:
- preparation of a first embryonated bird's egg according to claim 3 with CTC isolated from a sample of said patient or animal at a time T1, and tumorigenesis study of the tumours that develop in this first embryonated egg,
- preparation of a second embryonated bird's egg according to claim 3 with CTC isolated from a sample of the same patient or animal at a time T2, and tumorigenesis study of the tumours that develop in this second embryonated egg,
- comparison of the tumorigenesis of tumours that have developed in the first and in the second embryonated eggs.

9. Method for screening therapeutic agents intended for the *in vivo* treatment of cancer, comprising:
- preparation of an embryonated egg model using the method according to claim 3,
- administration of one or more candidate agents in the embryonated egg at least 12 hours after the graft,
- study of the effect of the therapeutic agent(s) thereby administered on the tumorigenesis of tumours that have developed in the grafted embryonated egg.
